# EUROPEAN PATENT APPLICATION

(11) **EP 3 476 341 A1**
(43) Date of publication of application: **01.05.2019**
(21) Application number: 18200672.6
(22) Date of filing: 16.10.2018
(51) Int. Cl.: A61B 17/80, A61B 5/00, A61B 8/08

(54) **CHECKING APPARATUS FOR BONE CONGLUTINATION**

(30) Priority: 24.10.2017 KR 20170138615
(71) Applicant: Paik, Hae Sun, Seoul 03075 (KR)
(72) Inventor: Paik, Hae Sun, Seoul 03075 (KR)
(74) Representative: Sanger, Phillip Simon

(57) **Abstract**

Provided is a checking apparatus for bone conglutination including a fixing plate (10) connecting an osteotomized bone; a sensor (20) provided on one surface of the fixing plate; and a driving device (30) driving the sensor after osteotomy, in which the fixing plate is coupled to the bone so that the sensor is positioned at an osteotomy region, and the sensor senses bone conglutination of the osteotomy region through contact with the bone filled at the osteotomy region when the sensor is driven.

## Description

### TECHNICAL FIELD

The present invention relates to a checking apparatus for bone conglutination capable of checking bone conglutination after osteotomy.

### BACKGROUND ART

In general, degenerative arthritis causes pains because the knee internal articular cartilage becomes severely worn to become a bent leg. In the degenerative arthritis, the progression and pains of the arthritis become severe when the body weight is concentrated at one side when standing or walking. There is proximal tibial osteotomy as a surgical method of this internal deformity. The proximal tibial osteotomy is a treatment method in which an abnormal axis of the lower limb is corrected to transfer the load applied to the knee joint to a healthier joint surface, thereby improving the distribution of stress and joint alignment and reducing pains. The proximal tibial osteotomy performs an operation of correcting a bent leg by cutting a thick bone below the knee, in which the calf bone is cut at a position close to the knee, the bone is opened at a required angle at the inside using a surgical instrument, the bone graft is performed, and then a fixation tool (Korean Patent Registration No. 10-1740905 "Fixation tool for opened proximal tibia osteotomy") formed of a metal plate is fixed using a screw. After the osteotomy, the bone conglutination needs to be checked. Bone grafting needs to be performed if the bone conglutination is not achieved. However, there was no method other than X-ray or computed tomography (CT) as a checking method for bone conglutination after the existing tibial osteotomy. In the X-ray or CT, imaging may be inconvenient and may be a burden on patients in cost. In addition, X-ray and CT are the most commonly used imaging devices, but there is a risk of repetitive radiation exposure caused by imaging.

Therefore, it is urgent to develop a device that can easily check the bone conglutination.

### SUMMARY OF THE INVENTION

The present invention has been made in an effort to provide a checking apparatus for bone conglutination capable of easily checking bone conglutination at an osteotomy region through a sensor provided in a fixing plate.

An exemplary embodiment of the present invention provides a checking apparatus for bone conglutination including a fixing plate connecting an osteotomized bone; and a sensor provided on one surface of the fixing plate, in which the fixing plate may be coupled to the bone so that the sensor is positioned at an osteotomy region, and the sensor may sense bone conglutination of the osteotomy region through contact with the bone filled at the osteotomy region when the sensor is driven.

The sensor may be a piezoelectric sensor.

The sensor may be a non-power sensor.

The checking apparatus for bone conglutination may further include a driving device, in which the driving device may be a conductor, and the conductor may generate an electric field or a magnetic field outside the skin of the patient after osteotomy, drive the sensor, and determine the bone conglutination by pressure information sensed by the sensor by applying the pressure to the sensor while the bone is filled in the osteotomy region.

According to the exemplary embodiment of the present invention, in the checking apparatus for bone conglutination, it is possible to easily check bone conglutination at an osteotomy region through a sensor provided in the fixing plate.

It is possible to reduce inconvenience and a burden in cost of X-ray or CT imaging.

It is possible to avoid a risk of radiation exposure due to X-ray and CT imaging.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

FIG. 1 is a view illustrating a state where a fixing plate having a sensor is coupled to a tibia according to a preferred exemplary embodiment of the present invention.
FIG. 2 is a view illustrating a process of driving a sensor by a driving device according to a preferred exemplary embodiment of the present invention.

It should be understood that the appended drawings are not necessarily to scale, presenting a somewhat simplified representation of various features illustrative of the basic principles of the invention. The specific design features of the present invention as disclosed herein, including, for example, specific dimensions, orientations, locations, and shapes will be determined in part by the particular intended application and use environment.

In the figures, reference numbers refer to the same or equivalent parts of the present invention throughout the several figures of the drawing.

### DETAILED DESCRIPTION

Hereinafter, preferred exemplary embodiments of the present invention will be described in detail with reference to the accompanying drawings. When reference numerals refer to components of each drawing, it is to be noted that although the same components are illustrated in different drawings, the same components are denoted by the same reference numerals as possible. Hereinafter, a preferred exemplary embodiment of the present invention will be described, but the technical spirit of the present invention is not limited or restricted thereto and the present invention may be modified and variously implemented by those skilled in the art.

First, a configuration of a checking apparatus for bone conglutination according to an exemplary embodiment of the present invention will be described.

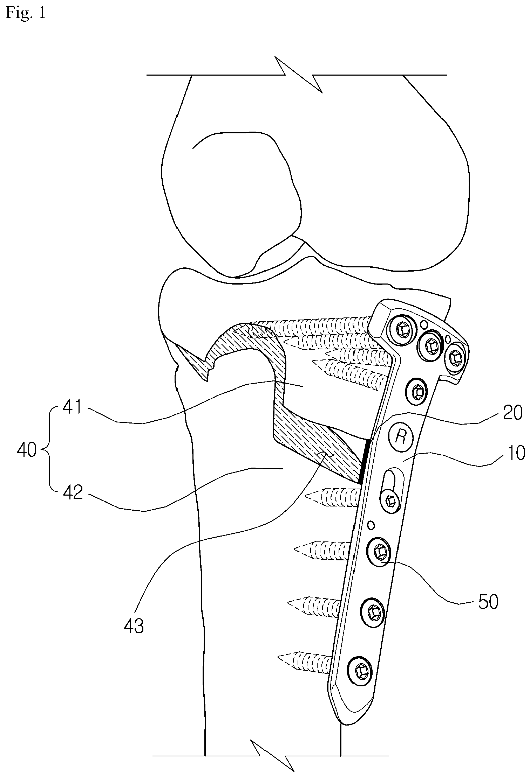

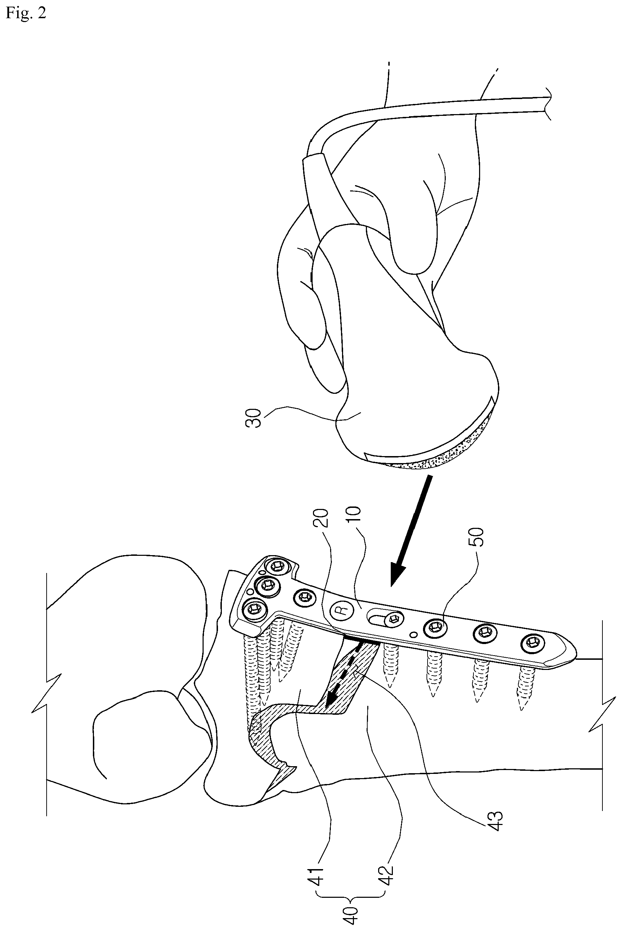

As illustrated in FIGS. 1 and 2, a checking apparatus for bone conglutination according to an exemplary embodiment of the present invention includes a fixing plate 10, a sensor 20 attached to one surface of the fixing plate 10, and a driving device 30 driving the sensor 20.

An osteotomy region 43 is formed in a tibia 40 by osteotomy. The tibia 40 is divided into an upper tibia 41 and a lower tibia 42 based on the osteotomy region 43. The fixing plate 10 connects the upper tibia 41 and the lower tibia 42. The fixing plate 10 may be a metal plate. The fixing plate 10 is coupled to the upper tibia 41 and the lower tibia 42 by a screw 50.

The sensor 20 may be a non-power piezoelectric sensor. The sensor 20 is provided on one surface of the fixing plate 10. The sensor 20 is positioned in a direction of the tibia 40. During osteotomy, the sensor 20 is positioned at the osteotomy region 43. The sensor 20 senses the pressing pressure of the bone while being in contact with the bone when the bone is filled in the osteotomy region 43.

For example, the sensor 20 may be designed as a wire sensor having a power source, but in this case, unnecessary power may be supplied to the sensor 20 regardless of a desired operation timing of the sensor 20, and there may be various inconveniences such as exposure of the wire connected to the sensor 20 to the outside of the skin. Therefore, the sensor 20 is designed as a non-power sensor to prevent inconvenience after skin sealing.

Core Chips Co., Ltd. (http://nano_korea.blog.me/80137472507) is known as a developer of non-power wireless pressure sensor, and the non-power sensor technology is a well-known technology and the detailed description thereof is omitted.

The driving device 30 drives the sensor 20 outside the skin. The driving device 30 may be a conductor. The driving device 30 generates an electric field or a magnetic field to drive the sensor 20. The driving device 30 is not limited in structure and form. The driving device 30 may be any device capable of generating an electric field or a magnetic field to drive the sensor 20. For example, the driving device 30 may be a hand-held doppler, a duplex scan, or the like of a medical ultrasound testing apparatus. Since the driving device 30 for generating the electric field or the magnetic field to drive the sensor 20 is a general device, the detailed description thereof will be omitted.

Next, a process of determining bone conglutination of the checking apparatus for bone conglutination according to the exemplary embodiment of the present invention will be described.

As illustrated in FIG. 2, the osteotomy region 43 is formed by cutting the tibia 40 according to opened proximal tibial osteotomy. The tibia 40 is divided into the upper tibia 41 and the lower tibia 42 based on the osteotomy region 43.

After the upper tibia 41 and the lower tibia 42 which have been bent are corrected, the sensor 20 is positioned at the osteotomy region 43 and the upper portion of the fixing plate 10 is in contact with the upper tibia 41 and the lower tibia 42 so that the screw 50 is fastened to the upper tibia 41 and the lower tibia 42 through a screw hole of the fixing plate 10.

The fixing plate 10 serves to support a load applied to the tibia 40 until the bone conglutination of the upper tibia 41 and the lower tibia 42 is achieved. After the proximal tibial osteotomy, it is required to check the conglutination of the bone. The reason is that bone grafting needs to be performed when the bone conglutination is not achieved.

After the opened proximal tibial osteotomy, the bone conglutination is checked. Specifically, the driving device 30 generates an electric field or a magnetic field to drive the sensor 20 outside the sutured skin of the patient. Since the sensor 20 is driven only by the driving device 30, unnecessary driving of the sensor 20 may be prevented. The sensor 20 driven by the driving device 30 senses the pressing pressure of the bone while being in contact with the bone filled in the osteotomy region 43. The bone conglutination may be determined based on the pressure information sensed by the sensor 20. For example, when a pressure value sensed by the sensor 20 is equal to or greater than a set value, it may be determined as the bone conglutination.

As described above, since the checking apparatus for bone conglutination according to the exemplary embodiment of the present invention can easily check the bone conglutination at the osteotomy region through the sensor provided on the fixing plate, it is possible to completely solve problems such as inconvenience of imaging, the burden in cost, and the risk of radiation exposure caused when determining bone conglutination by existing X-ray or CT.

The above description is only illustrative of the technical spirit of the present invention, and it will be apparent to those skilled in the art that various modifications, substitutions and alterations can be made hereto without departing from the spirit and scope of the invention as defined by the appended claims. Therefore, the exemplary embodiments of the present invention and the accompanying drawings are provided for illustrative purposes only but not intended to limit the technical concept of the present invention, and the scope of the technical concept of the present invention is not limited thereto according to the exemplary embodiments of the present invention and the accompanying drawings. The protective scope of the present invention should be construed based on the appended claims, and all the technical spirits in the equivalent scope thereof should be construed as falling within the scope of the present invention.

As described above, the exemplary embodiments have been described and illustrated in the drawings and the specification. The exemplary embodiments were chosen and described in order to explain certain principles of the invention and their practical application, to thereby enable others skilled in the art to make and utilize various exemplary embodiments of the present invention, as well as various alternatives and modifications thereof. As it is evident from the foregoing description, certain aspects of the present invention are not limited by the particular details of the examples illustrated herein, and it is therefore contemplated that other modifications and applications, or equivalents thereof, will occur to those skilled in the art. Many changes, modifications, variations and other uses and applications of the present construction will, however, become apparent to those skilled in the art after considering the specification and the accompanying drawings. All such changes, modifications, variations and other uses and applications which do not depart from the spirit and scope of the invention are deemed to be covered by the invention which is limited only by the claims which follow.

## Claims

1. A checking apparatus for bone conglutination comprising:
a fixing plate connecting an osteotomized bone; and
a sensor provided on one surface of the fixing plate,
wherein the fixing plate is coupled to the bone so that the sensor is positioned at an osteotomy region, and the sensor senses bone conglutination of the osteotomy region through contact with the bone filled at the osteotomy region when the sensor is driven.

2. The checking apparatus for bone conglutination of claim 1, wherein the sensor is a piezoelectric sensor.

3. The checking apparatus for bone conglutination of claim 1, wherein the sensor is a non-power sensor.

4. The checking apparatus for bone conglutination of claim 1, further comprising:
a driving device,
wherein the driving device is a conductor, and the conductor generates an electric field or a magnetic field outside the skin of a patient after osteotomy, drives the sensor, and determines the bone conglutination by pressure information sensed by the sensor by applying the pressure to the sensor while the bone is filled in the osteotomy region.
